# EUROPEAN PATENT SPECIFICATION

(11) **EP 2 320 919 B1**
(45) Date of publication and mention of the grant of the patent: **30.03.2016**
(21) Application number: 09798201.1
(22) Date of filing: 24.06.2009
(51) Int. Cl.: A61K 36/00, A61K 36/18, A61P 3/04, A61K 127/00

(54) **LARGE-SCALE PROCESS FOR THE PREPARATION OF THYLAKOIDS**
VERFAHREN IM GROSSEN MASSSTAB ZUR HERSTELLUNG VON THYLAKOIDEN
PROCESSUS À GRANDE ÉCHELLE POUR LA PRÉPARATION DE THYLACOÏDES

(30) Priority: 14.07.2008 SE 0801682
(43) Date of publication of application: 18.05.2011
(73) Proprietor: THYLABISCO AB, 224 71 Lund (SE)
(72) Inventor: ERLANSON-ALBERTSSON, Charlotte, S-224 71 Lund (SE); ALBERTSSON, Per-Åke, S-224 71 Lund (SE); LILIUS, Gösta, S-247 92 Södra Sandby (SE); OLBE, Malin, S-247 92 Södra Sandby (SE)
(74) Representative: Brann AB
(86) International application number: PCT/SE2009/000327
(87) International publication number: WO 2010/008333

(56) References cited:
- WO-A1-2005/027944
- WO-A1-2006/132586
- WO-A1-2008/019920
- KOHNKE R ET AL: "Thylakoids promote release of the satiety hormone cholecystokinin while reducing insulin in healthy humans", SCANDINAVIAN JOURNAL OF GASTROENTEROLOGY, INFORMA HEALTHCARE, GB, vol. 44, no. 6, 1 January 2009 (2009-01-01), pages 712-719, XP009159372, ISSN: 0036-5521, DOI: 10.1080/00365520902803499
- RICKARD KÖHNKE ET AL: "Thylakoids Suppress Appetite by Increasing Cholecystokinin Resulting in Lower Food Intake and Body Weight in High-fat Fed Mice", PHYTOTHERAPY RESEARCH, JOHN WILEY & SONS LTD. CHICHESTER, GB, [Online] vol. 23, no. 12, 1 December 2009 (2009-12-01), pages 1778-1783, XP002676081, ISSN: 0951-418X, DOI: 10.1002/PTR.2855 Retrieved from the Internet: URL:http://onlinelibrary.wiley.com/doi/10. 1002/ptr.2855/abstract> [retrieved on 2009-06-22]
- EMEK SINAN C ET AL: "A large scale method for preparation of plant thylakoids for use in body weight regulation.", PREPARATIVE BIOCHEMISTRY & BIOTECHNOLOGY 2010 LNKD- PUBMED:20024791, vol. 40, no. 1, 2010, pages 13-27, XP9162696, ISSN: 1532-2297
- HINCHA D.K.: 'Effects of calcium-induced aggregation on the physical stability of liposomes containing plant glycolipids' BIOCHIMICA ET BIOPHYSICA ACTA vol. 1611, no. 1-2, 2003, pages 180 - 186, XP004416804
- ALBERTSSON P.-A. ET AL: 'Chloroplast membranes retard fat digestion and induce satiety: Effect of biological membranes on pancreatic lipase/co-lipase' BIOCHEMICAL JOURNAL vol. 401, no. 3, 2007, ISSN 0264-6021 pages 727 - 733, XP003026055
- CASAZZA A.P. ET AL: 'Preparation and functional characterization of thylakoids from Arabidopsis thaliana' PHOTOSYNTHESIS RESEARCH vol. 68, no. 2, 2001, ISSN 0166-8595 pages 175 - 180, XP019263579

## Description

### FIELD OF INVENTION

The present invention relates to a process on a large industrial scale for the production of thylakoids, from photosynthetic organisms, such as from green plant leaf material, to be used as ingredients in food, or additions to food, or dietary supplements, or pharmaceuticals for the purpose of preventing overweight, promoting satiety, reducing food intake, reducing bodyweight, reducing blood insulin concentration and reducing blood fats and percentage body fat in humans and animals.

### BACKGROUND OF INVENTION

Overweight and obesity has become a world-wide problem. Obesity, being a part of the metabolic syndrome, can lead to several diseases such as diabetes, arteriosclerosis and hypertension. Obesity and overweight are the result of an imbalance between energy intake and energy expenditure and is often a result of lack of appetite control. High-fat diet, either alone or together with sucrose and other sweet carbohydrates, is one of the most important factors causing obesity since these diets easily promote overeating. In the present invention we have also found that the preparation of thylakoids described here have similar effects in humans. For application on humans a large-scale preparation is required.

Thylakoids have so far been prepared on a small laboratory scale in much the same way as used in research on photosynthesis using media with a composition to preserve the integrity of the thylakoid function as active in biological electron transport and with preserved redox systems.

However, to be able to produce the thylakoids in large scales you are faced with a number of problems including maintaining the proteins intact, i.e., they should not be degraded. Furthermore unwanted components in the leaf cells need to be efficiently removed eliminating or substantially removing the unwanted components from the final product.

### SUMMARY OF THE INVENTION

The present invention relates to a large-scale process suitable for industrial production for the preparation of a membrane fraction comprising thylakoids. Such a process is required for administration of thylakoids to humans and animals. Several effects of thylakoids on humans related to promoting satiety and preventing overweight, are described in the invention. There are a number of problems when scaling up a process wherein the process concerns plant material. As for the plant material, plant cells are hard to break due to the rigid cell walls covering the cells. Also, plant materials that have been grown outdoors in field have been exposed to different kinds of microorganisms as well as different weather conditions which are unable to control. Additionally, unwanted compounds such as e.g. heavy metals are taken up by the roots of the plants, which may influence the process and the final product. Problems which never occur when the plant material is grown under controlled condition in a greenhouse. When the plant material is harvested it is highly important that all the processes within the plant are stopped and that the microorganisms are somehow inactivated. If the metabolism in the plants is not stopped degradation of the plant material will occur and make a process for the isolation of thylakoids impossible. The purpose of the present invention is to rapidly and efficiently isolate the thylakoids and to maintain the structure of the thylakoids, wherein the proteins are intact and use the thylakoids as ingredients in food or additions to food, or dietary supplements or pharmaceuticals for promoting satiety, retarding fat digestion, reduce blood insulin and decrease blood fatty acids such as triglycerides and body fat in humans and animals.

In a first aspect the invention relates to a large-scale process for the production of a membrane fraction comprising the steps of collecting thylakoid containing plants or algae in an amount larger of 1000 or more kg, homogenising said plants or algae with a solution containing for example EDTA into a homogenate, removing plant debris from the homogenate, collecting the thylakoids in a fraction, concentrating the fraction, washing the fraction with ethanol and optionally drying the concentrate fraction to a powder or granulate.

### BRIEF DESCRIPTION OF DRAWINGS

Fig. 1. Precipitation of thylakoids as a function of pH. Suspensions of thylakoids at the same concentration but at different pH were allowed to stand in a container for one hour. The height of the liquid was 20 cm. The ordinate shows how much of the thylakoids that remain in the supernatant as measured by absorbance at 680 nm (a wavelength where thylakoids have maximum absorbance). This demonstrates that the precipitation is effective in the pH range 4 to 5. pH was adjusted by addition of HCl to a suspension having a pH of 7.
Fig. 2. Processing of green leaves into thylakoids. Part I. 1. Washing, freezing and storage of plant material; 2. Mixing, homogenisation and chelating steps; 3. Dilution and separation of plant debris.
   1; 1.1. Washing, 1.2. Freezing, 1.3, Cutter, 1.4 Packing for storage, 1.5 Storage
   2; 2.1 Mixing, 2.2 Homogenising, 2.3 Chelator
   3; 3.1 Dilution, 3.2 Separation by decanter
Fig. 3. Processing of green leaves into thylakoids. Part II. 4. A. Isolation of thylakoids by separator;. B. Isolation of thylakoids by pH precipitation; 5. A+B. Treatment with ethanol; 6. Drying.
   4A; 4.1. Separator, 4.2 Precipitation, 4.3 Filtration.
   4B; 4.1. pH precipitation, 4.2.1. Freezing, 4.2.2. Dekanter. 4.3 Filtration.
   5A,B; 5.1.Ethanol treatment.
   6. Drying proceedures.
Fig. 4. Insulin and glucose levels after a meal with and without thylkaoids in human. As seen there was a significant reduction of insulin secretion (left) by thylakoids and no change on glucose levels (right).
Fig. 5. Effect of thylakoids on food intake (A), body weight (B) and body fat (C) in mice fed either a high-fat diet (control) or a high-fat diet enriched with thylakoids (thylakoids) for 100 days. Food intake (A), body weight (B) and body fat (C) was significantly reduced in the thylakoid-treated animals.

### DETAILED DESCRIPTION OF THE INVENTION

### Definitions

In the context of the present application and invention, the following definitions apply:
The term "thylakoids or thylakoid membranes" is intended to mean the photosynthetic membranes in any photosynthetic organism, such as from plants.
The term "leaves" is intended to mean any green leaves from plants.
The term "homogenising" is intended to mean any kind of mixing, milling, cutting that reduces the size of the material, such as the leaf material as well as divide, disrupt, dissolve the photosynthetic material into smaller parts, even breaks the cells of the material, such as a plant material.
The term "cutter" is intended to mean a device that can cut (milling) fresh or frozen photosynthetic material into smaller fragments.
The term "disintegrator" is intended to mean a device that can disrupt or dissolve the cells of the material, such as the cells of the leaves.
The term "slurry" is intended to mean the product after disintegrating the photosynthetic material and mixing it with a solvent medium.
The term "decanter" is intended to mean a device that can, in a continuous process remove large debris, such as cell walls, fragments of cell walls, unbroken cells, starch grains, from a suspension containing thylakoids obtained after disruption of the photosynthetic material.
The term "separator" is intended to mean a device that can separate and collect thylakoids from their surrounding liquid, on a large scale by centrifugation in a continuous process.
The term "chelating agent" is intended to mean an agent that can by binding to heavy metals remove these from the thylakoids.
The term "agent" is intended to comprise the term chelating agent above. "Agent" may also refer to a protease inhibitor.
The term "large scale" is intended to mean a scale much larger than laboratory scale, e.g. 1 000 kg or more of photosynthetic material is used as the starting material for preparation of thylakoids. The photosynthetic material may be a plant material.

### The large-scale process

The invention relates to a large-scale process to release the thylakoids from any photosynthetic organism such as leaf materials. Such leaf material may be obtained from any source of green leaves such as spinach, clover, rape, sugar beet, kale, quinoa, grass, Jerusalem artichoke, dandelion, Chenopodium, Atriplex. The material may be obtained from crops grown on free land or in green houses.

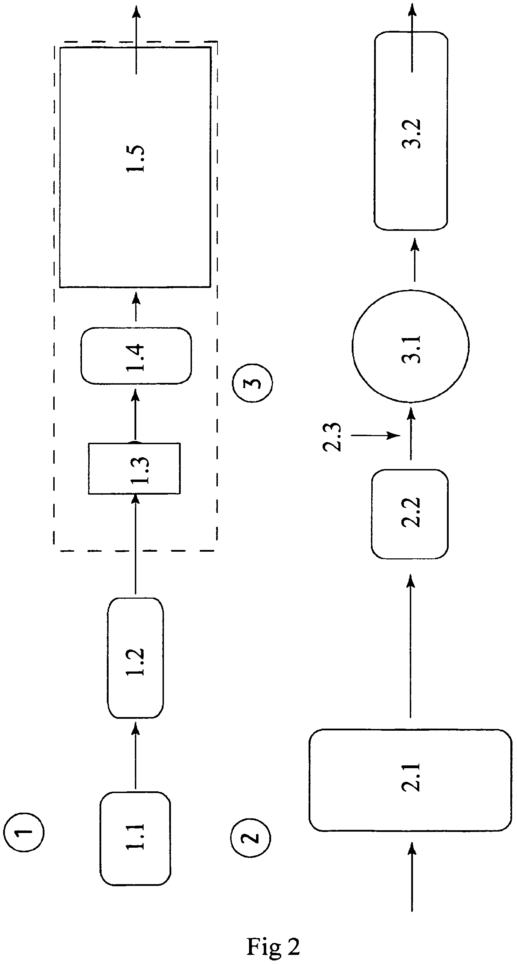

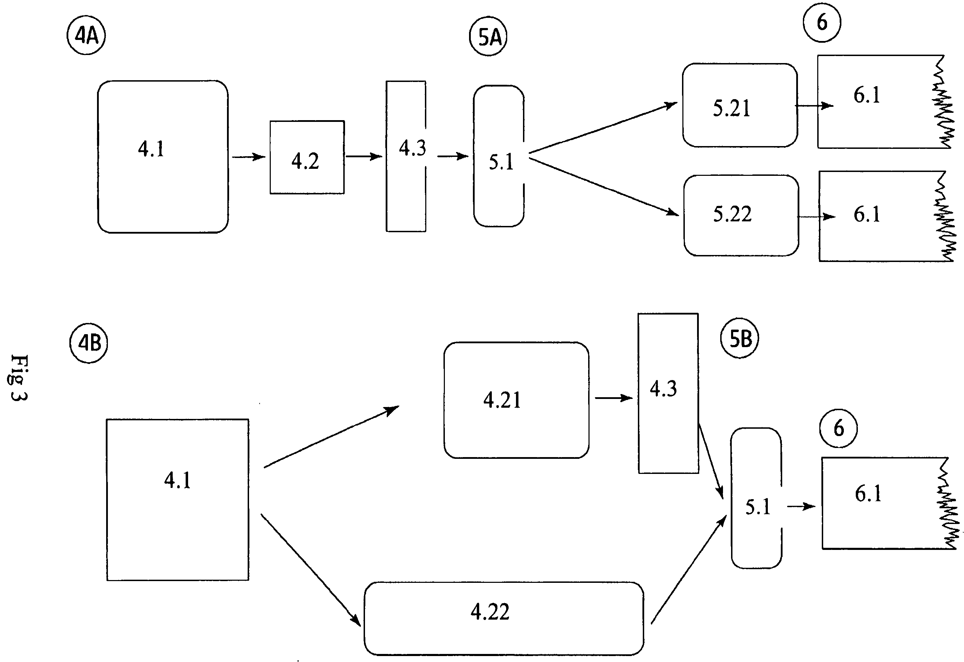

The process comprises a number of steps such as those mentioned below (Fig. 2, Fig. 3). All the steps are not necessary and may be included depending on the starting material such as which plant as well as what the isolated thylakoids should be used for as well as where the plants have been grown.

### Harvesting/collecting and rapid freezing of the leaf material

The leaf material is either processed as fresh material, or rapidly frozen by a method that minimises the degradation of proteins present within the leaves as well as prohibiting the growth of microorganisms, such as bacteria and moulds. The freezing step may be performed in a freezing tunnel with cold air that is produced by a conventional freezing unit (-40°C) and/or by the use of liquid nitrogen (-196°C). As a next step the leaf material may be homogenised into smaller pieces by the use of a cutter, such as a conventional mixer (Fig. 2). Example is a mill such as a hammer mill. The division of the leaf material prior to storage is performed to reduce the volume of the frozen material. Further by the use of a first freezing step the ice crystals formed during the freezing process puncture the leaf cells, which simplify the proceeding steps. The leaf material may then be stored in a frozen stabilised form for further use or processed immediately.

### Disintegrating the homogenate/slurry and treatment by a chelator

In the homogenising process the leaf material is mixed with a liquid such as water for example drinking water, such as 1 part of leaf material and 1-5 parts of water into a slurry/homogenate. Other examples include 1 part of leaf material and 1, 2, 3, 4 or 5 parts of water. The slurry/homogenate will then be transferred into a disintegrator such as pumped into a mill, such as mechanically pressed, such as ultra-sonicated, which disrupts, dissolves or breaks the cells of the plant. The leaves and the leaf cells can also be exposed to added enzyme macerases such as cellulase, hemicellulase and pectinase either alone or in combination to dissolve or weaken the cell walls in order to facilitate the disintegration process.

The disintegrating step releases the thylakoids into the solution of the slurry/homogenate.
The slurry/homogenate is then treated by a metal chelator 0.5-50 mM, such as 0.5-40 mM, 0.5-30 mM, 0.1-20mM, 0.5-10 mM or 8-12 mM, such as EDTA, EGTA, DTPA, citrate and NTA. Such chelators remove or reduce the heavy metals which are bound to the thylakoids.

Addition of a metal chelator inhibits aggregation of the thylakoid membranes. This facilitates later steps in the process, in particular the use of a separator, and ultimately leads to a purer product. Addition of a metal chelator also inhibits metal-dependent lipoxygenases, which protects the product from unwanted degradation and oxidation of fatty acids. Also, addition of a metal chelator inhibits metal-dependent proteases, which protects the product from unwanted degradation of proteins.

Other chelators, agents, or combination of agents which achieve some or all of the benefits mentioned above are also included in the invention. For example, one may include protease inhibitors, chelators or a combination thereof.

Optionally, divalent ions such as calcium and/or magnesium ions may be added back to the product in a later step in the large-scale process.

Table 1. Effect of EDTA on the content (micrograms/gram dry weight) of heavy metals in thylakoids.

EDTA at a concentration of 10 mM was included in the extaction medium during disintegration of the leaf cells.

The heavy metals were determined by atomic absorption spectroscopy. 0.5 gram dry thylakoids were dissolved in nitric acid and deionized water (7-3mL) in a micro-oven. The analysis was carried out with ICP MS (model Elan 6000 from Perkin Elmer) and ICP AES (model OPTIMA 3000 DV from Perkin-Elmer). Thyl EDTA = Thylakoids treated with EDTA; Control = thylakoids without treatment with EDTA.

| | **Al** | **Cd** | **Cu** | **Fe** | **Pb** | **Sn** | **Zn** |
|---|---|---|---|---|---|---|---|
| **Thyl EDTA** | 396 | 0,17 | 3,4 | 417 | 0,23 | 0,27 | 5,10 |
| | | | | | | | |
| **Control** | 3829 | 2,19 | 15,9 | 3834 | 5,03 | 2.0 | 54 |

The following steps are different steps of isolation of the thylakoid membranes which may be used alone or in combination.

### Dilution of the slurry/homogenate and removal of plant debris

After the above defined steps the slurry/homogenate comprises the plant debris as well as the cytoplasm. The cytoplasm comprises the different organelles. The slurry will be further diluted with an aqueous solution such as drinking water. For example, the slurry may be diluted to a final ratio of plant material and water, such as drinking water of 1:5, 1:6, 1:7, 1:9 or 1:10 or even more to achieve a washing step. The plant debris is removed from the slurry by for example a decanter or filtration. The decanter separates the cell parts from the solution.

### Isolation of the thylakoides, concentration and wash

The isolation step may be performed in a number of ways such as using a separator or pH-precipitation as long as the result is the concentration or isolation of the thylakoids.

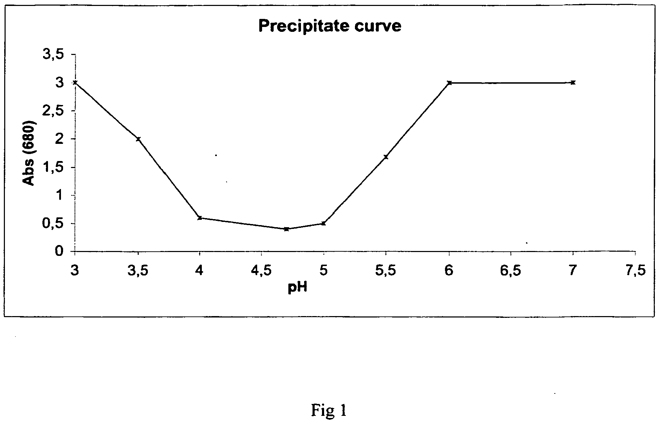

In one embodiment (Fig. 3. 4A) the use of a separator with a suitably high G-number isolates the thylakoid membranes from the slurry. The volume of the isolated thylakoid fraction will be reduced about 5, 10, 15, 20 or even more times. To be able to concentrate the thylakoid fraction even further it is possible to reduce the pH to between 4 and 5, preferably about 4.7 (Fig. 1). At this pH the thylakoids form a thick flowing liquid phase which is easy to collect by filtration, sedimentation or centrifugation. By this embodiment it is possible to create a fraction of thylakoids having a high purity since the soluble proteins have been removed.

In another embodiment (Fig. 3. 4B) it is possible to isolate the thylakoids by pH-precipitation, after removing the plant debris as for example with a decanter. This may be performed by lowering the pH to between 4 and 5, preferably about 4.7 (Fig. 1), as above and then isolating the thylakoid fraction from the slurry without using a separator.

In a third embodiment the thylakoids may be concentrated by filtration immediately after the step of using a separator, by using for example a mesh having a pore size of 5 µm which allows collection of the thylakoid membranes. By such a step it is possible to obtain a protein fraction with about 25 % dry weight after filtration.

If there is a need the thylakoids may be further concentrated by a step of freeze-thawing whereby the liquid is separated from the remaining thylakoids.

Additional step of washing the thylakoid fraction may be necessary depending of which plant material the thylakoid membranes are isolated from or depending on what the product will be used for. Such a washing step may include dilution of the thylakoid membranes in drinking water.

### 5. Additional treatment of the thylakoid membranes

The thylakoid membranes may be treated with any organic solvent to prevent growth of micororganisms, such as treated with between 20 % to 30 % ethanol, such as at least 22% ethanol, for example 22 % to 25% ethanol, for example 22 % ethanol. The lower percentage will be used to prevent growth of microorganisms and a higher percentage is used for the delipidation of thylakoids to get a pure protein fraction (Fig. 3). Other treatments may be the addition of taste increasing ingredients, or aromas or other suitable agents, concentrating the product such as by removing the lipids.

### 6. Drying of the thylakoid membranes

An additional step of drying the thylakoid membranes as a step of isolating the thylakoid membranes may be included. Different ways of drying may be used as long as the thylakoid membranes maintain their structure. Examples of drying include spray drying, air drying, freeze drying, drying with carbon dioxide as well as vacuum drying.

The isolated thylakoid membrane fraction obtained by the method/process mentioned above may be used as an additive and may be admixed with other components such as fat, butter, margarine, oils, cream, milk, cheese, brie, flour, juices, soft drinks, teas or any other suitable product. Other examples of products are ice cream, yoghurt, cakes, bread, cereals, pasta and dressing. The thylakoid membranes may be sold in sachets to be added to any product prior to consumption. Other examples of products are mentioned in PCT/SE2006/00676.

The invented composition may be used as a food additive and may be admixed with other components such as fat, butter, margarine, oils, cream, milk, cheese, brie, flour, juices, soft drinks, teas either prior to being added to a food product or during the addition to the food product. Said food additive or food composition comprising said composition may be solid, semisolid or in a liquid form. Further it may be freeze dried, spray dried or lyophilised. The invented food additive may be used in any kind of food product as well as being used alone. Examples of food products are fat, butter, margarine, oils, cream, milk, cheese, brie, flour, juices, soft drinks, teas. Other examples are yoghurt, ice cream, cakes, bread and dressing.

The invented composition may also be used as a pharmaceutical composition and the invention also relates to the use of the product that can be obtained by the method above or a method that gives the same product for the manufacturing of a medicament for the treatment of overweight, promoting satiety, reducing food intake, reducing bodyweight, reducing blood insulin concentration and reducing percentage body fat in humans and animals.

The pharmaceutical composition comprises the invented composition as well as a pharmaceutically acceptable buffer, excipient, carrier or diluent. Examples of diseases to be treated are the metabolic syndrome either as a disease or a disorder such as hypertension, arteriosclerosis, gout, diabetes type one and two, cancers and dyslipidemia. "Pharmaceutically acceptable" means a non-toxic material that does not decrease the effectiveness of the biological activity of the active ingredients, i.e., the antimicrobial peptide(s). Such pharmaceutically acceptable buffers, carriers or excipients are well-known in the art (see Remington's Pharmaceutical Sciences, 18th edition, A.R Gennaro, Ed., Mack Publishing Company (1990) and handbook of Pharmaceutical Excipients, 3rd edition, A. Kibbe, Ed., Pharmaceutical Press (2000).

The term "buffer" is intended to mean an aqueous solution containing an acid-base mixture with the purpose of stabilising pH. Examples of buffers are Trizma, Bicine, Tricine, MOPS, MOPSO, MOBS, Tris, Hepes, HEPBS, MES, phosphate, carbonate, acetate, citrate, glycolate, lactate, borate, ACES, ADA, tartrate, AMP, AMPD, AMPSO, BES, CABS, cacodylate, CHES, DIPSO, EPPS, ethanolamine, glycine, HEPPSO, imidazole, imidazolelactic acid, PIPES, SSC, SSPE, POPSO, TAPS, TABS, TAPSO and TES.

The term "diluent" is intended to mean an aqueous or non-aqueous solution with the purpose of diluting the peptide in the pharmaceutical preparation. The diluent may be one or more of saline, water, polyethylene glycol, propylene glycol, ethanol or oils (such as safflower oil, corn oil, peanut oil, cottonseed oil or sesame oil).

The term "adjuvant" is intended to mean any compound added to the formulation to increase the biological effect of the peptide. The adjuvant may be one or more of zinc, copper or silver salts with different anions, for example, but not limited to fluoride, chloride, bromide, iodide, tiocyanate, sulfite, hydroxide, phosphate, carbonate, lactate, glycolate, citrate, borate, tartrate, and acetates of different acyl composition. The excipient may be one or more of carbohydrates, polymers, lipids and minerals. Examples of carbohydrates include lactose, sucrose, mannitol, and cyclodextrines, which are added to the composition, e.g., for facilitating lyophilisation. Examples of polymers are starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone, all of different molecular weight, which are added to the composition, e.g., for viscosity control, for achieving bioadhesion, or for protecting the lipid from chemical and proteolytic degradation. Examples of lipids are fatty acids, phospholipids, mono-, di-, and triglycerides, ceramides, sphingolipids and glycolipids, all of different acyl chain length and saturation, egg lecithin, soy lecithin, hydrogenated egg and soy lecithin, which are added to the composition for reasons similar to those for polymers. Examples of minerals are talc, magnesium oxide, zinc oxide and titanium oxide, which are added to the composition to obtain benefits such as reduction of liquid accumulation or advantageous pigment properties.

The compositions of the invention may also be in the form of polymer gels, where polymers such as starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone are used for thickening of the solution containing the peptide.

The pharmaceutical compositions may be subjected to conventional pharmaceutical operations such as sterilisation and/or may contain conventional adjuvants such as preservatives, stabilisers, wetting agents, emulsifiers, buffers, fillers, etc., e.g., as disclosed elsewhere herein.

Suitable preparation forms are, for example granules, powders, tablets, coated tablets, (micro) capsules, syrups, emulsions, microemulsions, defined as optically isotropic thermodynamically stable systems consisting of water, oil and surfactant, liquid crystalline phases, defined as systems characterised by long-range order but short-range disorder (examples include lamellar, hexagonal and cubic phases, either water- or oil continuous), or their dispersed counterparts, gels, ointments, dispersions, suspensions, creams, aerosols, droplets or injectable solution in ampoule form and also preparations with protracted release of active compounds, in whose preparation excipients, diluents, adjuvants or carriers are customarily used as described above.

The pharmaceutical compositions will be administered to a patient in a pharmaceutically effective dose. By "pharmaceutically effective dose" is meant a dose that is sufficient to produce the desired effects in relation to the condition for which it is administered. The exact dose is dependent on the, activity of the compound, manner of administration, nature and severity of the disorder, age and body weight of the patient different doses may be needed. The administration of the dose can be carried out both by single administration in the form of an individual dose unit or else several smaller dose units and also by multiple administration of subdivided doses at specific intervals.

The present invention concerns both humans and other mammal such as horses, dogs, cats, cows, pigs, camels, among others. Thus the methods are applicable to both human therapy and veterinary applications. The objects, suitable for such a treatment may be identified by well-established hallmarks. Here follows, as an example of the invention, a description of the isolation of chloroplast membranes, the thylakoids, from spinach and their application in inhibition of the pancreatic lipase activity and reduction of food intake. Following examples are intended to illustrate, but not to limit, the invention in any manner, shape, or form, either explicitly or implicitly.

### EXAMPLES

### Example 1

### Production of thylakoids from spinach

Production of 30 kg dry thylakoid membrane fraction from spinach needs a starting material of about 1 000 kg.

Dry content spinach: 8-9%

About 0.01%-0.03% (w/w) of dry thylakoid membrane fraction were obtained from spinach fresh weight.
1. 1 000 kg frozen spinach was mixed with drinking water 1:1 and homogenised using a screw press and a blender. The amount of dry substance was about 85 kg.
2. EDTA was added to a concentration of 10 mM.
3. The homogenate/slurry was diluted to a final ratio of 1:10, with respect to spinach and drinking water, to a volume of 10 m³. The slurry was forced through a decanter whereby the cell debris was removed from the liquid.
4. The thylakoid fraction was isolated by an alfa laval separator. Around 600 liter thick liquid with a dry substance of about 5% was obtained.
5. After freezing at 24 hours, thawing and filtration through 5µm filter the thylakoid fraction was concentrated 5 times to a paste having a dry substance content of about 25%. From 1 000 kg spinach 120 kg paste was obtained.
6. The paste was dried by the use of a freeze dryer and about 30 kg dried thylakoid membranes were obtained.

### Example 2

### Effect of the product on insulin

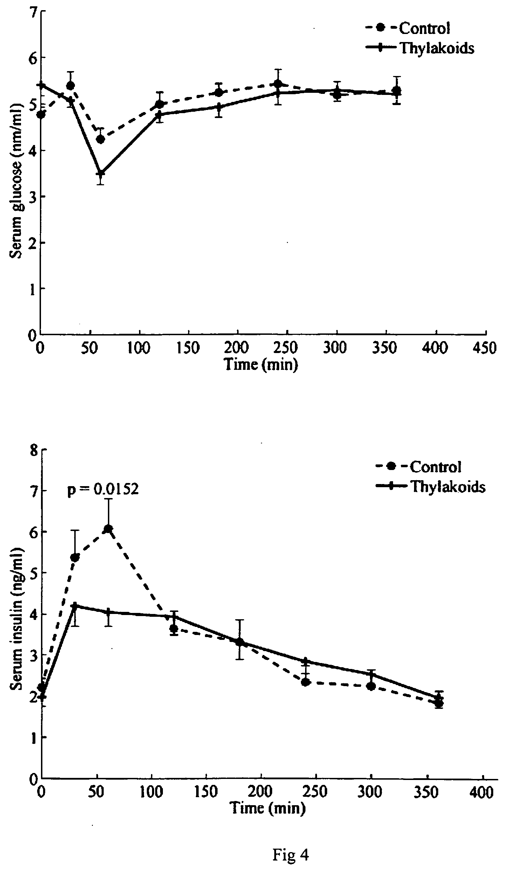

Insulin has an important anabolic effect, promoting overeating and obesity. In various diet regimens it is therefore important to achieve a reduction in insulin levels. To investigate if the thylakoids affects insulin secretion insulin was measured during intake of a control meal and a meal containing thylakoids. The study was performed in human. 11 healthy humans were fasted over night and then served either a control meal or a thylakoid enriched meal with a week between the meals to ensure a washout effect. The control meal contained bread, tomatoes and a pesto sauce containing rapeseed oil and cashew nuts spiced with basil, whereas the thylakoid meal (4 different experimental meals) contained the same components added with various doses of thylakoid powder (either 50 gram, 25 gram, 10 gram or 5 gram). Blood samples were taken at each time point 0, 30 min, 1 hour, 2 h, 4 h and 6 h. Glucose and insulin were measured. Thylakoids had no effect on blood glucose levels, whereas insulin secretion was significantly reduced at all doses. The significant reduction of insulin secretion suggests that insulin sensitivity was increased by the thylakoid powder. Such an effect is extremely important for establishing energy balance. The effect of thylakoids on insulin secretion is demonstrated for 50 gram thylakoids (Fig.4).

### Example 3

### Effect of the product on body fat

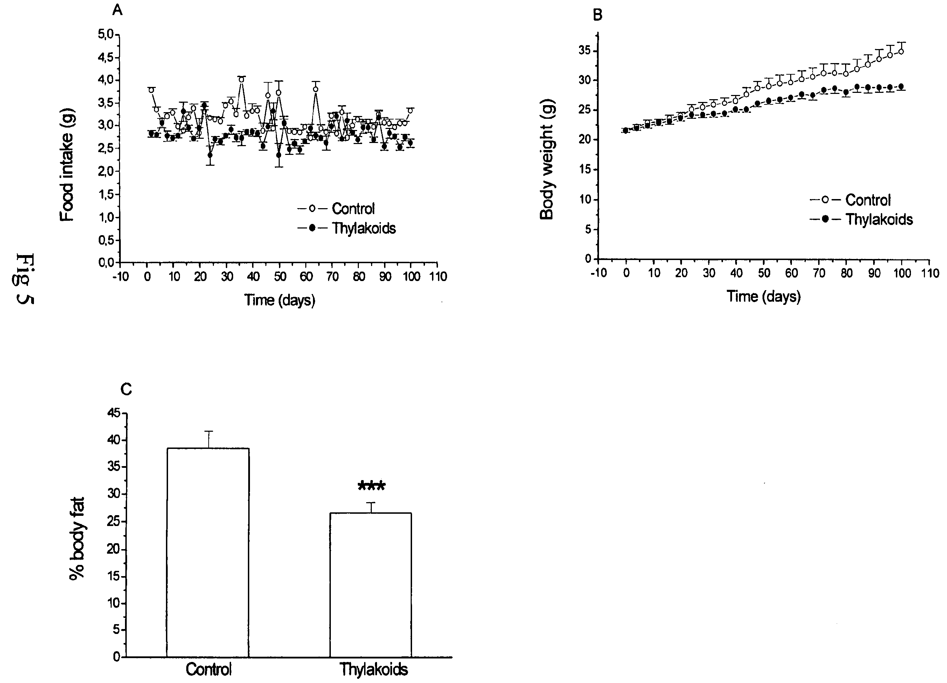

To study the effect of thylakoids on body fat mice were fed either a high-fat diet or a high-fat diet enriched with thylakoids for 100 days. Thirty female apoE-deficient mice were obtained from Taconic (Taconic Europe, Lille Skensved, Denmark) and divided into two groups (n=15 per group); one group receiving high-fat diet containing 0.15% cholesterol by energy and 43% saturated fat by energy (control group; R638, Lactamin, Kimsta, Sweden) and the other group receiving high-fat diet (R638) enriched with thylakoids, the dose of 6 mg chlorophyll/g diet was used. The control diet and the thylakoid enriched diet was isocaloric and with the same energy-composition between macronutrients. The mice were housed three per cage in a temperature-controlled environment (21°C), with a 12 h light/dark cycle (lights on at 6 a.m.). The mice had free access to their respective diets and tap water at all times. Food intake was measured every other day and body weight twice a week. The study lasted for 100 days. Finally, after four hours fasting blood was collected and the triglycerides was determined in the serum with a GPO-Trinder kit from Sigma (Sigma diagnostics, Steinheim, Gernany). The results are shown in the table below. As seen the triglycerides were significantly reduced in the animals receiving thylakoids compared to control animals .Then, the animals were killed and body fat analysed using dual energy X-ray analysis (DEXA).:As indicated in figure 5, food intake, body weight and body fat was significantly reduced in the animals receiving thylakoids compared to control animals (Fig. 5).

### Triglycerides (mmol/L)

| | Control | Tylakoids |
|---|---|---|
| mean | 1,21 | 0,93 |
| SD | 0,33 | 0,23 |

| | | |
|---|---|---|
| CD= standard deviation | | |

## Claims

1. A large-scale process for the production of at least a thylakoid membrane fraction comprising the steps of:
a) collecting at least thylakoid containing plants or algae in an amount of 1000 kg or more,
b) homogenising said plants or algae with a solution into a homogenate, wherein said solution in b) contains an agent selected from the group comprising EDTA, EGTA, DTPA, citrate and NTA,
c) removing plant debris from the homogenate of b),
d) collecting the thylakoids in a fraction,
e) concentrating the fraction of d) and
f) drying the concentrate fraction of e) to a powder or granulate.

2. The process according to claim 1, wherein the homogenising step b) is performed by the use of at least one mixing step, milling step or disintegrating step.

3. The process according to any of claims 1 to 2, wherein step d) comprises using a separator.

4. The process according to any of claims 1 to 2, wherein step d) comprises a precipitation step using a pH of about 4 to 5.

5. The process according to claims 1 to 4, wherein the concentrating step of e) is performed by centrifugation or filtration.

6. The process according to any one of claims 1 to 5, wherein a freezing step is used in between step d) and step e), to mechanically and/or enzymatically disintegrate the leaves.

7. The process according to any of claims 1 to 6, wherein said process comprises at least one washing step.

8. The process according to claim 7, wherein the washing step is performed after step e).

9. The process according to any one of claims 7 to 8 wherein the washing step comprises washing with ethanol.

10. The process according to any of preceding claims, wherein said photosynthetic organism is a plant selected from the group consisting of algae, spinach, clover, rape, sugar beet, kale, quinoa, grass or Jerusalem artichoke, dandelion, *Chenopodium*, *Atriplex.*

11. The process according to any of preceding claims, wherein said process comprises an additional step of adding said thylakoid membrane fraction to a food, dietary supplement or a pharmaceutical product.

## Patentansprüche

1. Großtechnisches Verfahren zur Erzeugung wenigstens einer Thylakoidmembranfraktion, umfassend die folgenden Schritte:
a) Sammeln von wenigstens Thylakoid beinhaltenden Pflanzen oder Algen in einer Menge von 1000 kg oder mehr,
b) Homogenisieren der Pflanzen oder Algen mit einer Lösung zu einem Homogenat, wobei die Lösung nach Schritt b) einen aus der aus EDTA, EGTA, DTPA, Citrat und NTA bestehenden Gruppe ausgewählten Wirkstoff umfasst,
c) Entfernen der Pflanzenreste aus dem Homogenat nach Schritt b),
d) Sammeln der Thylakoiden zu einer Fraktion,
e) Konzentrieren der Fraktion nach Schritt d) und
f) Trocknen der konzentrierten Fraktion nach Schritt e) zu einem Pulver oder Granulat.

2. Verfahren nach Anspruch 1, wobei der Homogenisierungsschritt b) unter der Anwendung wenigstens eines Mischungsschrittes, Mahlschrittes oder Zerkleinerungsschrittes ausgeführt wird.

3. Verfahren nach einem der Ansprüche 1 oder 2, wobei Schritt d) die Verwendung eines Abscheiders umfasst.

4. Verfahren nach einem der Ansprüche 1 oder 2, wobei Schritt d) einen Ausfällungsschritt unter der Verwendung eines pH-Wertes von 4 bis 5 umfasst.

5. Verfahren nach einem der Ansprüche 1 bis 4, wobei der Konzentrationsschritt nach Schritt e) durch Zentrifugierung oder Filtrierung ausgeführt wird.

6. Verfahren nach einem der Ansprüche 1 bis 5, wobei zur mechanischen und/oder enzymatischen Zerkleinerung der Blätter zwischen den Schritten d) und e) ein Einfrierungsschritt verwendet wird.

7. Verfahren nach einem der Ansprüche 1 bis 6, wobei das Verfahren wenigstens einen Auswaschungsschritt umfasst.

8. Verfahren nach Anspruch 7, wobei der Auswaschungsschritt nach Schritt e) ausgeführt wird.

9. Verfahren nach einem der Ansprüche 7 oder 8, wobei der Auswaschungsschritt die Auswaschung mit Ethanol umfasst.

10. Verfahren nach einem der vorgehenden Ansprüche, wobei es sich bei dem photosynthetischen Organismus um eine aus der aus Algen, Spinat, Klee, Raps, Zuckerrübe, Kohl, Quinoa, Artischocke oder Jerusalem-Artischocke, Löwenzahn, Gänsefüßen und Melden bestehenden Gruppe ausgewählte Pflanze handelt.

11. Verfahren nach einem der vorhergehenden Ansprüche, wobei das Verfahren einen zusätzlichen Schritt der Hinzufügung der Thylakoidmembranfraktion zu einem Lebensmittel, einer Beikost oder einem pharmazeutischen Produkt umfasst.

## Revendications

1. Processus à grande échelle destiné à la production d'au moins une fraction de membrane de thylakoïde comprenant les étapes de :
a) collecte d'au moins des plantes ou des algues contenant des thylakoïdes dans une quantité de 1 000 kg ou plus,
b) homogénéisation desdites plantes ou algues avec une solution en un homogénat, dans lequel ladite solution en b) contient un agent choisi dans le groupe comprenant l'EDTA, l'EGTA, le DTPA, du citrate et le NTA,
c) élimination des débris de plante de l'homogénat de b),
d) collecte des thylakoïdes d'une fraction,
e) concentration de la fraction de d) et
f) séchage de la fraction de concentré de e) en une poudre ou des granulés.

2. Processus selon la revendication 1, dans lequel l'étape b) d'homogénéisation est réalisée en utilisant au moins une étape de mélange, une étape de broyage ou une étape de désintégration.

3. Processus selon l'une quelconque des revendications 1 à 2, dans lequel l'étape d) comprend l'utilisation d'un séparateur.

4. Processus selon l'une quelconque des revendications 1 à 2, dans lequel l'étape d) comprend une étape de précipitation en utilisant un pH d'environ 4 à 5.

5. Processus selon les revendications 1 à 4, dans lequel l'étape de concentration de e) est réalisée par centrifugation ou filtration.

6. Processus selon l'une quelconque des revendications 1 à 5, dans lequel une étape de congélation est utilisée entre l'étape d) et l'étape e), afin de désintégrer mécaniquement ou/et enzymatiquement les feuilles.

7. Processus selon l'une quelconque des revendications 1 à 6, dans lequel ledit processus comprend au moins une étape de lavage.

8. Processus selon la revendication 7, dans lequel l'étape de lavage est réalisée après l'étape e).

9. Processus selon l'une quelconque des revendications 7 à 8, dans lequel l'étape de lavage comprend un lavage avec de l'éthanol.

10. Processus selon l'une quelconque des revendications précédentes, dans lequel ledit organisme photosynthétique est une plante choisie dans le groupe consistant en les algues, les épinards, le trèfle, le colza, la betterave sucrière, le chou frisé, le quinoa, l'herbe ou le topinambour, le pissenlit, le chénopode, *Atriplex.*

11. Processus selon l'une quelconque des revendications précédentes, dans lequel ledit processus comprend une étape supplémentaire d'addition de ladite fraction de membrane de thylakoïde à un aliment, un complément alimentaire ou un produit pharmaceutique.
